# EUROPÄISCHE PATENTANMELDUNG

(11) **EP 1 598 573 A1**
(43) Veröffentlichungstag der Anmeldung: **23.11.2005**
(21) Anmeldenummer: 05010462.9
(22) Anmeldetag: 13.05.2005
(51) Int. Cl.: F16F 9/53, F16F 9/46, F16F 9/26

(54) **Regelbarer Bewegungsdämpfer**

(30) Priorität: 19.05.2004 DE 202004008024 U
(71) Anmelder: Bauerfeind AG, 07937 Zeulenroda (DE)
(72) Erfinder: Reinhardt, Holger, 47906 Kempen (DE); Schönemann, Hans, 97616 Bad Neustadt (DE)
(74) Vertreter: Bardehle, Heinz

(57) **Zusammenfassung**

Die Erfindung betrifft einen regelbaren Bewegungsdämpfer (4) mit einem Dämpfungskolben (10'), der in einem Dämpfungszylinder (10) zwei Dämpfungsräume (12,13) gegeneinander abgrenzt, und einem in einem Verbindungskanal (28,29) der beiden Dämpfungsräume angeordneten Dämpfungsventil (15), dessen Durchlässigkeit für ein im Bewegungsdämpfer enthaltenes Dämpfungsfluid von einem Steuerorgan (21,16) gesteuert wird. Beide Dämpfungsräume sind mit einem elektromagnetisch unbeeinflussbaren Dämpfungsfluid gefüllt und das Steuerorgan weist eine mit einer magnetorheologischen Flüssigkeit gefüllte Schubkolbeneinheit (21) mit einem kraftschlüssig mit dem Dämpfungskolben gekoppelten Schubkolben (22) auf, der über die durch steuerbare Magnetfelderzeuger (30) hinsichtlich ihres Strömungswiderstandes beeinflussbare magnetorheologische Flüssigkeit auf ein Stellglied (16) wirkt, das mit dem Dämpfungsventil verbunden ist.

## Beschreibung

Die Erfindung bezieht sich auf einen regelbaren Bewegungsdämpfer mit einem Dämpfungskolben, der in einem Dämpfungszylinder zwei Dämpfungsräume gegeneinander abgrenzt, und einem in einem Verbindungskanal der beiden Dämpfungsräume angeordneten Dämpfungsventil, dessen Durchlässigkeit für ein im Bewegungsdämpfer enthaltenes Dämpfungsfluid von einem Steuerorgan gesteuert wird.

Ein derartiger regelbarer Bewegungsdämpfer für ein Prothesen-Kniegelenk ist aus der DE-OS 10214357 bekannt. Der Dämpfungszylinder dieses Bewegungsdämpfers ist mit einer als Dämpfungsfluid dienenden magnetorheologischen Flüssigkeit gefüllt, die von dem im Dämpfungszylinder axial beweglichen Dämpfungskolben bei dessen Bewegung verschoben wird, wobei die magnetorheologische Flüssigkeit ein Dämpfungsventil durchströmt, das die beiden durch den Dämpfungskolben getrennten Zylinderräume im Dämpfungszylinder verbindet. Je nach Durchlässigkeit des Dämpfungsventils sind entsprechend größere oder kleinere auf den Dämpfungskolben wirkende Kräfte erforderlich, um den Dämpfungskolben zu verschieben und damit die gewünschte Dämpfungswirkung auszuüben. Bei dem bekannten Bewegungsdämpfer dient als Steuerorgan für die Durchlässigkeit des Dämpfungsventils ein oder mehrere Elektromagnete, die durch ein zu höherer oder niedrigerer Intensität gesteuertes Magnetfeld in bekannter Weise die Viskosität der magnetorheologischen Flüssigkeit und damit ihren Strömungswiderstand im Dämpfungszylinder entsprechend verändern. In der genannten Druckschrift sind zwei Ausführungsbeispiele für die Anordnung des Dämpfungsventils vorgesehen. Gemäß einer Ausführungsform besteht das Dämpfungsventil aus einem Spalt zwischen dem Dämpfungsventil und dem Dämpfungskolben, wobei der Spalt durch ein steuerbares Magnetfeld durchsetzt wird. Gemäß einer weiteren Ausführungsform sind die beiden durch den Dämpfungskolben getrennten Dämpfungsräume über einen Nebenschluss verbunden, in dem bei Bewegung des Dämpfungskolbens die magnetorheologische Flüssigkeit strömt. Der Nebenschluss ist durch einen Magnetfelderzeuger geführt, dessen steuerbares Magnetfeld die Viskosität der im Bereich des Magnetfeldes strömenden magnetorheologischen Flüssigkeit in der jeweils gewünschten Weise zu geringerer oder stärkerer Dämpfung entsprechend steuert.

Ein weiterhin bekannter Bewegungsdämpfer ist in der EP 0957838 B1 beschrieben, der ebenfalls als Dämpfungsfluid eine magnetorheologische Flüssigkeit enthält.

Bei der in den bekannten Bewegungsdämpfern verwendeten magnetorheologischen Flüssigkeit (MRF) handelt es sich um ein Medium, das wegen seiner Füllung mit kleinen magnetisierbaren Teilchen ein hohes spezifisches Gewicht im Bereich von etwa 3 besitzt, das damit nahe dem halben spezifischen Gewicht von Eisen liegt. Infolgedessen wird in Anbetracht der erforderlichen Füllmengen des Bewegungsdämpfers dieser sehr schwer, was für seine praktische Handhabung einen erheblichen Nachteil darstellt. Vor allem lässt sich die Viskosität einer MRF nicht beliebig hoch einstellen. Das heißt, es tritt Sättigung ein. Dies bedeutet, dass trotz höherer magnetischer Leistung keine erhöhte Dämpfung mehr erzielt werden kann.

Es ist weiterhin aus der internationalen Anmeldung WO 99/27273 ein Bewegungsdämpfer bekannt, bei dem zwecks Verringerung der Menge einer magnetorheologischen Flüssigkeit in dem Dämpfer in dessen einem Dämpfungsraum eine übliche Dämpfungsflüssigkeit, z.B. Öl, und in dem anderen Dämpfungsraum die magnetorheologische Flüssigkeit untergebracht ist, die dabei aber auch noch als Dämpfungsflüssigkeit wirkt und damit einen erheblichen Anteil an dem Gewicht des Bewegungsdämpfers ausmacht.

Darüber hinaus sind Bewegungsdämpfer für das Prothesen-Kniegelenk bekannt, in denen nur eine übliche Dämpfungsflüssigkeit, also insbesondere Öl, verwendet wird (z.B. US 6,113,642), bei denen zur Steuerung des Durchflusses die Dämpfungsflüssigkeit elektromagnetisch betätigte Schieber als Dämpfungsventile verwendet werden, für deren Betätigung erhebliche elektrische Ströme notwendig sind, die eine entsprechend starke Stromversorgung des Dämpfers erforderlich machen.

Derartige Bewegungsdämpfer für Beinprothesen werden durch Programme gesteuert, die in elektronischen Steuerungen gespeichert sind. Ein Beispiel hierfür ist die GB 2328160 A.

Der Erfindung liegt die Aufgabe zugrunde, den eigentlichen Bewegungsdämpfer mit Dämpfungszylinder, Dämpfungskolben und Dämpfungsventil mit kleinem Leistungsgewicht, stromsparend und reaktionsschnell zu gestalten.

Erfindungsgemäß geschieht dies im Zusammenhang mit dem eingangs angegebenen Bewegungsdämpfer dadurch, dass beide Dämpfungsräume mit einem elektromagnetisch unbeeinflussbaren Dämpfungsfluid gefüllt sind und das Steuerorgan eine mit einer magnetorheologischen Flüssigkeit gefüllte Schubkolbeneinheit mit einem kraftschlüssig mit dem Dämpfungskolben gekoppelten Schubkolben aufweist, der über die durch einen steuerbaren Magneten hinsichtlich ihres Strömungswiderstandes beeinflussbare magnetorheologische Flüssigkeit auf ein Stellglied wirkt, das mit dem Dämpfungsventil verbunden ist.

In diesem derart gestalteten Bewegungsdämpfer ist in dem gleichen Dämpfungsbereich keinerlei magnetorheologische Flüssigkeit enthalten, so dass der betreffende Bereich des Bewegungsdämpfers hinsichtlich seines Gewichts gegenüber den bekannten ähnlichen Bewegungsdämpfern weitgehend entlastet ist, da er nur ein normales Dämpfungsfluid enthält, also Dämpfungsöl oder Gas, insbesondere Luft. Die in dem Steuerorgan enthaltene magnetorheologische Flüssigkeit beschränkt sich dabei im Vergleich zu dem Raumbedarf des eigentlichen Bewegungsdämpfers auf einen vergleichbar kleinen Raum, der lediglich der Füllung des Steuerorgans dient, bei dem es sich aber nicht um eine nennenswerte Dämpfungswirkung, sondern lediglich um eine Übertragung von geringen Kräften handelt, mit denen sich das Dämpfungsventil, das für den Grad der Dämpfung des Bewegungsdämpfers zuständig ist, leicht steuern lässt.

Darüber hinaus ergibt sich aufgrund der Freihaltung des Bewegungsdämpfers von der Verwendung einer magnetorheologischen Flüssigkeit der wesentliche Vorteil, dass sonst durch diese Flüssigkeit bewirkte Verschleißerscheinungen, die auf die der Flüssigkeit beigemengten magnetisierbaren Teilchen zurückzuführen sind, stark reduziert werden und die natürlich sich dann besonders auswirken, wenn in dem Bewegungsdämpfer erhebliche Kräfte aufzufangen sind, wie dies z.B. bei einem Prothesenkniegelenk der Fall ist. Dieser Verschleiß wirkt sich vor allem im Bereich von notwendigen Dichtungselementen aus, die bei Verwendung von üblichem Dämpfungsöl diesbezüglich keinerlei Verschleiß aufweisen, da sie durch das Dämpfungsöl geschmiert werden.

Bei dem erfindungsgemäßen Bewegungsdämpfer ist die magnetorheologische Flüssigkeit auf die Schubkolbeneinheit konzentriert, die aber einerseits räumlich einen geringen Umfang ausmacht, also hinsichtlich ihres Gewichtes nicht übermäßig in Erscheinung tritt. Außerdem werden in der Schubkolbeneinheit nur relativ geringe Kräfte übertragen, nämlich die auf das Dämpfungsventil wirkenden Kräfte, das aber für eine Verstellung keinerlei besonderen Kraftaufwand erfordert, was sich besonders verschleißmindernd auswirkt. Die hierfür erforderlichen Kräfte werden von dem Dämpfungskolben gewissermaßen abgezweigt, da der Dämpfungskolben kraftschlüssig mit dem Schubkolben der Schubkolbeneinheit gekoppelt ist, so dass also für die Betätigung der Schubkolbeneinheit und des von ihr gesteuerten Dämpfungsventils kein zusätzlicher Krafterzeuger erforderlich ist.

Eine günstige Steuerung der Schubkolbeneinheit lässt sich dadurch herbeiführen, dass die Schubkolbeneinheit zwei durch den Schubkolben getrennte Stellräume mit einem diese verbindenden Nebenschluss enthält, dessen Strömungswiderstand durch die Magnetfelderzeuger einstellbar ist. Über den Nebenschluss lässt sich die Einwirkung der Schubkolbeneinheit auf das Stellglied mittels des auf den Nebenschluss einwirkenden Magnetfelderzeugers in vorteilhafter Weise gestalten.

Zweckmäßig wird das Dämpfungsventil mit einem das Stellglied bildenden, von einer Rückstellfeder geführten Stößel verbunden, der mit einer Zwischenwand verstellbar verbunden ist, die die Schubkolbeneinheit in die zwei über den Nebenschluss verbundene Stellräume aufteilt. Dies hat zur Folge, dass nach jeder Bewegungsphase das Stellglied und damit das Dämpfungsventil wieder in seine Normallage zurückkehrt, aus der dann diese beiden Bestandteile des Bewegungsdämpfers je nach Art, insbesondere Schnelligkeit, einer neu einsetzenden Bewegungsphase verstellt werden, um auf diese Phase die gewünschte Einwirkung herbeizuführen.

Eine übersichtliche Anordnung der Schubkolbeneinheit in Bezug auf den Dämpfungszylinder ergibt sich dann, wenn das Dämpfungsventil neben dem Dämpfungszylinder und die Schubkolbeneinheit koaxial zum Dämpfungszylinder angeordnet ist. Es ist aber auch möglich, das Dämpfungsventil koaxial zum Dämpfungskolben als Bestandteil des Dämpfungskolbens anzuordnen, womit sich eine besonders kompakte Gestaltung ergibt.

Für die Gestaltung des Bewegungsdämpfers bietet sich einerseits ein in einem Dämpfungszylinder koaxial angeordneter und bewegter Dämpfungskolben an, wie eine solche Anordnung z.B. bei Automobil-Stoßdämpfern verwendet wird. Es ist aber auch möglich, den Bewegungsdämpfer und die Schubkolbeneinheit jeweils als Drehkolben zu gestalten und in jeweils einer sektorial verlaufenden Wand das Dämpfungsventil und den Nebenschluss vorzusehen, wobei das Dämpfungsventil als Drehventil ausgebildet ist und der Nebenschluss einen Drehflügel als Bestandteil des Stellglieds enthält und Drehventil und Drehflügel über eine Drehachse miteinander verbunden sind und durch eine Drehfeder in Normallage gehalten werden. Hierbei handelt es sich also im Wesentlichen um eine rotationssymmetrische Anordnung, die ebenfalls günstige Anbringungsmöglichkeiten bietet.

Eine besondere Möglichkeit der Steuerung des Dämpfungsventils besteht darin, dass das Dämpfungsventil über einen Steuerkolben mit dem Stellglied verbunden ist, das in seiner Schubkolbeneinheit mehrere, jeweils durch Magnetfelderzeuger einstellbare Nebenschlüsse enthält, die in Abhängigkeit von der Aktivierung der einzelnen Magnetfelderzeuger den Steuerkolben in beliebige Stellungen, insbesondere der Bewegung des Schubkolbens folgend, einstellen. Mit dieser wahlweise und unabhängig von einem Rückstellglied steuerbaren Schubkolbeneinheit lässt sich durch eine entsprechende Einstellung der Magnetfelderzeuger jede gewünschte Steuerung des Dämpfungsventils herbeiführen, ebenso latente Dämpfungseinstellungen bei bewegtem Schubkolben und inaktivem Magnetfelderzeuger, wobei diese Steuerung z.B. über Sensorik von einem Programm eines Kleinrechners stammen kann, das für bestimmte Bewegungsabläufe der zu dämpfenden Bewegung, z.B. medizinisch notwendige Bewegungsdämpfungen, gestaltet ist.

Anstelle der magnetorheologischen Flüssigkeit für die Betätigung des Steuerorgans kann auch eine äquivalent wirkende Flüssigkeit, nämlich eine elektrorheologische Flüssigkeit verwendet werden, die durch ein elektrisches Feld hinsichtlich ihrer Viskosität beeinflusst wird.

Es sei noch darauf hingewiesen, dass der erfindungsgemäße regelbare Bewegungsdämpfer nicht nur in Prothesen, insbesondere Beinprothesen verwendbar ist, sondern auch in anderen Fällen, in denen es sich um die Bedämpfung einer hin- und hergehenden Bewegung, wie dies bei einem Stoßdämpfer der Fall ist, handelt,

In den Figuren sind Ausführungsbeispiele der Erfindung dargestellt. Es zeigen:
- Fig. 1: eine Beinprothese mit einem regelbaren Bewegungsdämpfer in der gestreckten Lage der Beinprothese;
- Fig. 2: die gleiche Beinprothese in Beugestellung des das Knie ersetzenden Teils der Beinprothese;
- Fig. 3: eine Gestaltung des Bewegungsdämpfers, bei dem das Dämpfungsventil neben dem Dämpfungszylinder angeordnet ist;
- Fig. 4: eine Gestaltung des Bewegungsdämpfers, bei dem das Dämpfungsventil koaxial zum Dämpfungskolben angeordnet ist;
- Fig. 5a-e: die Gestaltung eines Bewegungsdämpfers, bei dem der Bewegungsdämpfer und die Schubkolbeneinheit jeweils als Drehkolbenzylinder gestaltet sind;
- Fig. 6: eine Gestaltung des Bewegungsdämpfers, bei dem das Dämpfungsventil wahlweise in beliebige Stellungen einstellbar ist.

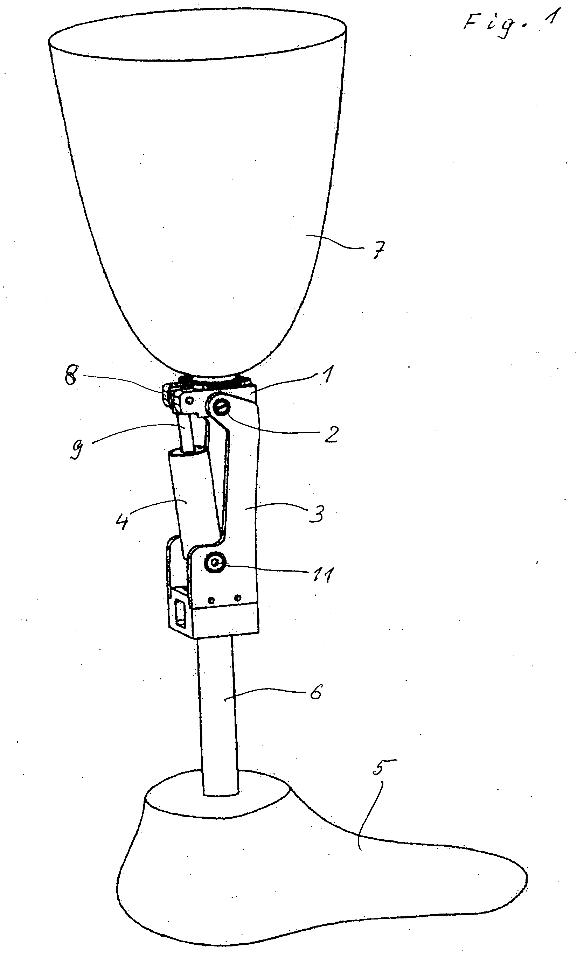

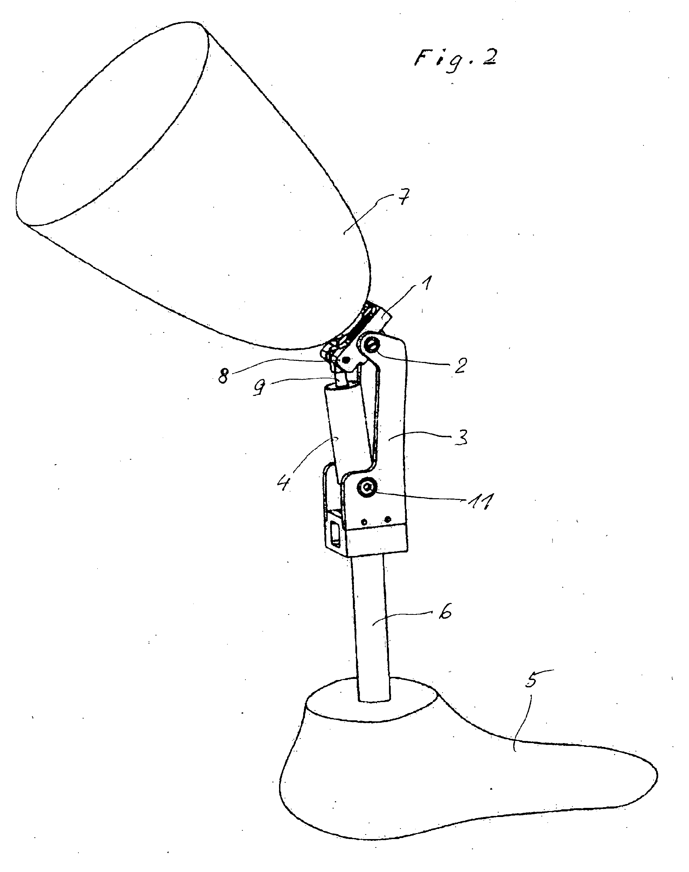

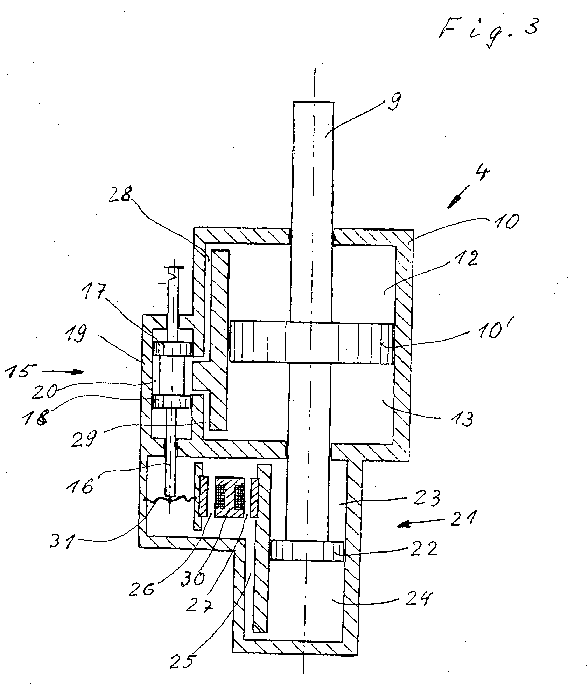

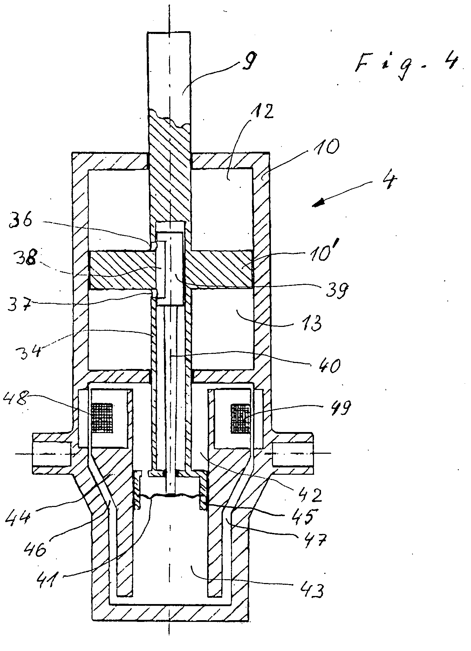

Bei der in den Figuren 1 und 2 schematisch dargestellten Beinprothese handelt es sich um eine solche mit dem künstlichen Kniegelenk 1, das um die Achse 2 drehbar ist, die das obere Unterschenkelteil 3 durchsetzt, an dem in Richtung zum Fußteil 5 hin das untere Unterschenkelteil 6 als Verbindung zwischen dem oberen Unterschenkelteil 3 und dem Fußteil 5 angeordnet ist. An dem Kniegelenk 1 ist in Richtung weg vom Unterschenkelteil 3 das Oberschenkelteil 7 befestigt, das gleichzeitig zur Aufnahme eines Oberschenkelstumpfes dient. Bei der Beugung des Kniegelenks 1 bewegt sich sein Hebelarm 8 in Richtung zum Fußteil 5 (siehe Fig. 2), wobei es den Dämpfungskolbenstößel 9 eines in den Figuren 3 und 4 dargestellten Bewegungsdämpfers entsprechend verschiebt. Dieser in den Figuren 1 und 2 mit 4 bezeichnete Bewegungsdämpfer dämpft die Kniegelenkbewegungen gemäß einem gewünschten Programm, wobei sich der Bewegungsdämpfer 4 in seiner Lage zum oberen Unterschenkelteil 3 geringfügig verschiebt. Zur Ermöglichung dieser Verschiebung ist der Bewegungsdämpfer 4 an seiner dem Fußteil 5 zugewandten Seite auf der Achse 11 gelagert. Die Steuerung des Bewegungsdämpfers 4 mittels eines Programms ist nicht Gegenstand dieser Erfindung. Hierzu sei beispielsweise auf die oben erwähnte GB 2328160 verwiesen.

Ausgehend von der in Fig. 1 dargestellten gestreckten Lage der Beinprothese lässt sich also das künstliche Kniegelenk 1 um die Achse 2 drehen und nimmt bei etwa halb gebeugtem Kniegelenk 1 die in der Fig. 2 dargestellte Lage ein, in der der Dämpfungskolben 10' (siehe Fig. 3 und 4) gegenüber seiner in der Fig. 1 dargestellten Lage weiter in den Bewegungsdämpfer 4 eingeschoben ist.

Insoweit handelt es sich prinzipiell um eine bekannte Gestaltung, wie sie auch in der EP 0 857 838 dargestellt ist.

In der Fig. 3 ist der in den Figuren 1 und 2 schematisch dargestellte Bewegungsdämpfer 4 hinsichtlich seines inneren Aufbaus dargestellt. Er weist den Dämpfungskolbenstößel 9 auf, der in dem Dämpfungszylinder 10 verschiebbar angeordnet ist und dabei den Dämpfungskolben 10' mitnimmt. Der Dämpfungskolben 10' teilt den Dämpfungszylinder 10 in die beiden Dämpfungsräume 12 und 13 auf, die mit einem Dämpfungsfluid, insbesondere einem üblichen Dämpfungsöl, gefüllt sind. Dabei kann es sich aber auch um ein Gas, z.B. Luft, handeln. Das Dämpfungsfluid wird bei der Bewegung des Dämpfungskolbens 10' verschoben, wobei sich eine entsprechende Strömung in dem Verbindungskanal, bestehend aus dem oberen Kanalteil 28 und dem unteren Kanalteil 29, ergibt. Beide Kanalteile 28 und 29 sind über das Dämpfungsventil 15 miteinander verbunden, das in seiner in der Fig. 3 dargestellten Lage die beiden Kanalteile 28 und 29 derart miteinander verbindet, dass bei der Verschiebung des Dämpfungskolbens 10' das Dämpfungsventil 15 einen praktisch strömungswiderstandslosen Druckausgleich zwischen den Dämpfungsräumen 12 und 13 ermöglicht.

Das Dämpfungsventil 15 enthält den als Stellglied für das Dämpfungsventil 15 wirkenden Stößel 16, der bei seiner Verschiebung die beiden Ventilteller 17 und 18 verschiebt, die in dem Dämpfungsventilzylinder 19 verschiebbar gelagert sind. In der in der Fig. 3 dargestellten Lage des Stößels 16 bildet der Zwischenraum 20 zwischen den beiden Ventiltellern 17 und 18 eine Strömungsverbindung für die beiden Kanalteile 28 und 29, so dass in der in der Fig. 3 dargestellten Lage des Dämpfungsventils 15 der Bewegungsdämpfer eine Verstellung des Dämpfungskolbens 10' praktisch unbedämpft belässt.

Wenn jedoch der Stößel 16 die beiden Ventilteller 17 und 18 verschiebt, so dass diese die in den Zwischenraum 20 endenden Kanalteile 28 und 29 mehr oder minder verschließen, so ergibt sich wegen des hierdurch bewirkten erhöhten Strömungswiderstandes in dem durch die Kanalteile 28 und 29 gebildeten Nebenschluss ein Widerstand gegen die Verschiebung des Dämpfungskolbens 10' und damit eine gewünschte Bedämpfung der Bewegung des Kniegelenks 1 (siehe Figuren 1 und 2). Auf diese eine Bedämpfung bewirkende Einstellung des Dämpfungsventils 15 wird nachstehend näher eingegangen.

Der Dämpfungskolbenstößel 9 ragt in die Schubkolbeneinheit 21 hinein und verschiebt in dieser den Schubkolben 22, der die Schubkolbeneinheit 21 in zwei getrennte Stellräume 23 und 24 aufteilt, die über den Nebenschluss 25 miteinander verbunden sind. Die Schubkolbeneinheit 21 und die mit den Stellräumen 23 und 24 verbundenen Räume sind mit einer magnetorheologischen Flüssigkeit gefüllt. Bei Verschiebung des Dämpfungskolbens 10' und damit des Schubkolbens 22 ergibt sich also in dem Nebenschluss 25 für die darin enthaltene magnetorheologische Flüssigkeit eine Strömung, die durch die beiden Teile 26 und 27 des Nebenschlusses 25 fließt. Strömungstechnisch parallel zu den Nebenschlussteilen 26 und 27 liegt die von einer Membranfeder gebildete biegeelastische Zwischenwand 31, die aufgrund ihrer Biegeelastizität als Rückstellfeder für den mit ihr verbundenen Stößel 16 wirkt. Aufgrund dieses Aufbaus ergibt sich bei der Bewegung des Dämpfungskolbens 10' folgende Funktion: der Dämpfungskolben 10' wirkt auf den Schubkolben 22, der dabei einen Druck bzw. Unterdruck auf die Stellräume 23 und 24 ausübt, wobei der jeweilige Druckunterschied über den Nebenschluss 25 ausgeglichen wird. Dabei ergibt sich zunächst auf den beiden Seiten der Zwischenwand 31 kein Druckunterschied, so dass damit die Lage des Stößels 16 unbeeinflusst bleibt. Wenn jedoch in den beiden Nebenschlussteilen 26 und 27 der Strömungswiderstand für die magnetorheologische Flüssigkeit erhöht wird, dann ergibt sich ein entsprechender Druck bzw. Unterdruck auf den beiden Seiten der Zwischenwand 31, die daraufhin entsprechend ausgelenkt wird und den Stößel 16 mitnimmt. Dieser verschiebt die Ventilteller 17 und 18, womit entweder der Ausgang des Kanalteils 28 oder des Kanalteils 29 verengt und damit der Verbindungskanal für die beiden Dämpfungsräume 12 und 13 verengt wird. Dies hat eine entsprechende Bedämpfung der Bewegung des Dämpfungskolbens 10' und damit eine Bedämpfung der Bewegung der in den Figuren 1 und 2 dargestellten Beinprothese zur Folge.

Die Beeinflussung der Viskosität der die beiden Nebenschlussteile 26 und 27 durchströmten magnetorheologischen Flüssigkeit erfolgt durch den Magnetfelderzeuger 30, bei dem es sich um eine von einem elektrischen Strom durchflossene Spule handelt, die ein von der jeweiligen Stromstärke abhängiges Magnetfeld erzeugt, das sich über die Nebenschlussteile 26 und 27 in bekannter Weise schließt. Mit stärker werdendem Magnetfeld in diesem Bereich wird die Viskosität der magnetorheologischen Flüssigkeit in Richtung stärkere Dickflüssigkeit verändert, womit sich auf beiden Seiten der Zwischenwand 31 entsprechende Druckunterschiede und damit eine entsprechende Verstellung des Dämpfungsventils 15 ergibt, die sich dann in einer entsprechenden Bedämpfung des Bewegungsdämpfers 9/10 äußert.

Aus den vorstehenden Darlegungen ergibt sich, dass der Bewegungsdämpfer, bestehend aus dem Dämpfungskolben 10' und dem Dämpfungszylinder 10 über das Dämpfungsventil 15 unter Benutzung eines üblichen Dämpfungsfluids wirksam ist, wobei aber die besonderen Vorteile einer auf elektrischen Signalen eines Programms beruhenden Steuerung der Dämpfung dieses Bewegungsdämpfers durch ein über eine magnetorheologische Flüssigkeit gesteuertes Dämpfungsventil in vorteilhafter Weise ausgenutzt werden, wobei die Energie für die Betätigung des Dämpfungsventils von der Bewegung des Dämpfungskolbens über die Schubkolbeneinheit abgezweigt wird, die aufgrund ihrer Füllung mit der magnetorheologischen Flüssigkeit die vorteilhafte Steuerung des Dämpfungsventils mittels elektrischer Signale ermöglicht.

Die in der Fig. 3 dargestellte Gestaltung des Bewegungsdämpfers weist eine Lage des Dämpfungsventils 15 auf, das seitlich neben dem Dämpfungszylinder 10 angeordnet ist. Demgegenüber handelt es sich bei dem Ausführungsbeispiel gemäß Fig. 4 um eine koaxial zum Dämpfungskolben angeordnete Lage des Dämpfungsventils, die gegenüber dem Ausführungsbeispiel gemäß Fig. 3 einen räumlichen Vorteil in Bezug auf die Unterbringung in einem Bewegungsdämpfer bietet, wie in den Figuren 1 und 2 gezeigt ist.

Eine solche koaxiale Lage ist in der Fig. 4 dargestellt. Bei dem Ausführungsbeispiel gemäß Fig. 4 weist der Bewegungsdämpfer, wie im Ausführungsbeispiel gemäß Fig. 3 den Dämpfungskolbenstößel 9 und den Dämpfungszylinder 10 auf, in dem der Dämpfungskolbenstößel 9 den Dämpfungskolben 10' verschiebt. Der Dämpfungskolbenstößel 9 enthält hier in seiner Verlängerung das Rohrstück 34, dessen Innenraum den Nebenschluss für die beiden Dämpfungsräume 12 und 13 bildet. Dieser Nebenschluss verläuft über die beiden Öffnungen 36 und 37 in dem Rohrstück 34, die nach innen zu in den Hohlraum 38 münden. In dem Rohrstück 34 ist das Ventilteil 39 verschiebbar gelagert, das den Hohlraum 38 umfasst und in der in der Fig. 4 dargestellten Lage die Dämpfungsräume 12 und 13 über die Öffnungen 36 und 37 miteinander verbindet. Bei einer Verschiebung des Ventilteils 39 werden dann die Öffnungen 36 bzw. 37 mehr oder minder abgesperrt, was der Verschiebung der Ventilteller 17 und 18 gemäß Fig. 3 entspricht.

Die Verschiebung des Ventilteils 39 erfolgt über den Stößel 40, der ähnlich wie bei dem Ausführungsbeispiel gemäß Fig. 3 von der als Membranfeder ausgebildeten Zwischenwand 41 verstellt wird. Die Zwischenwand 41 bildet eine biegeelastische Rückstellfeder für den Stößel 40 zwischen zwei Räumen 42 und 43 einer Schubkolbeneinheit, die durch den Zylinder 44 und den darin axial beweglichen Schubkolben 45 gebildet wird. Der Schubkolben 45 hängt an dem den Dämpfungskolbenstößel 9 verlängernden Rohrstück 34 und wird bei der Bewegung des Dämpfungskolbenstößels 9, also einer Bewegung des Kniegelenks, hin- und herbewegt. So lange der Stößel 40 an dieser Mitnahme durch den Dämpfungskolbenstößel 9 nicht gehindert wird, ergibt sich keine Verschiebung des Ventilteils 39 gegenüber den Öffnungen 37 und 38, so dass bei der gleichzeitigen Bewegung des Dämpfungskolbens 10' die in den beiden Dämpfungsräumen 12 und 13 enthaltene Dämpfungsflüssigkeit frei über den Hohlraum 38 strömen kann und damit kein eine Dämpfung bewirkender Druckunterschied in den beiden Dämpfungsräumen 12 und 13 entstehen kann. Die Bewegung des Dämpfungskolbenstößels 9 und damit der Kniegelenkprothese bleibt damit unbedämpft.

Bei dieser Bewegung des Stößels 40 ergibt sich auch eine entsprechende Verschiebung der Zwischenwand 41, die dabei die in den Räumen 42 und 43 enthaltene magnetorheologische Flüssigkeit verschiebt, was über die Nebenschlussteile 46 und 47 ungehindert vor sich geht, die die beiden Räume 42 und 43 miteinander verbinden. In diesen Nebenschlussteilen 46 und 47 sind nun, ähnlich wie bei dem Ausführungsbeispiel gemäß Fig. 3 Magnetfelderzeuger 48 und 49 untergebracht, die bei entsprechendem Stromdurchfluss und damit einem auf die Nebenschlussteile 46 und 47 wirkenden Magnetfeld die Viskosität der magnetorheologischen Flüssigkeit entsprechend beeinflussen und damit die Verschiebung der Zwischenwand 41 entsprechend behindern. Die Magnetfelderzeuger 48 und 49 können als Ringpolmagnet ausgebildet sein. Es ergibt sich damit eine Relativbewegung zwischen dem gewissermaßen festgehaltenen Stößel 40 und dem Schubkolben 45, was sich durch eine entsprechende Relativbewegung zwischen dem Ventilteil 39 und den Öffnungen 36 und 37 äußert. Damit ergibt sich eine Verstellung des das Ventilteil 39 enthaltenden Dämpfungsventils, womit die Bewegung des Stößels 9 und damit der Beinprothese entsprechend bedämpft wird. Funktionell stimmt also das Ausführungsbeispiel gemäß Fig. 4 mit demjenigen gemäß Fig. 3 überein, so dass diesbezüglich auf die Erläuterungen zu Fig. 3 verwiesen wird.

Der Vorteil der konstruktiven Gestaltung des Ausführungsbeispiels gemäß Fig. 4 besteht darin, dass bei diesem der Bewegungsdämpfer mit seinem Dämpfungsventil und dem zugehörigen Steuerorgan koaxial hintereinander angeordnet sind, wodurch sich die Unterbringung dieser Ausführungsform bei einem Bewegungsdämpfer, wie in den Figuren 1 und 2 dargestellt ist, vereinfacht.

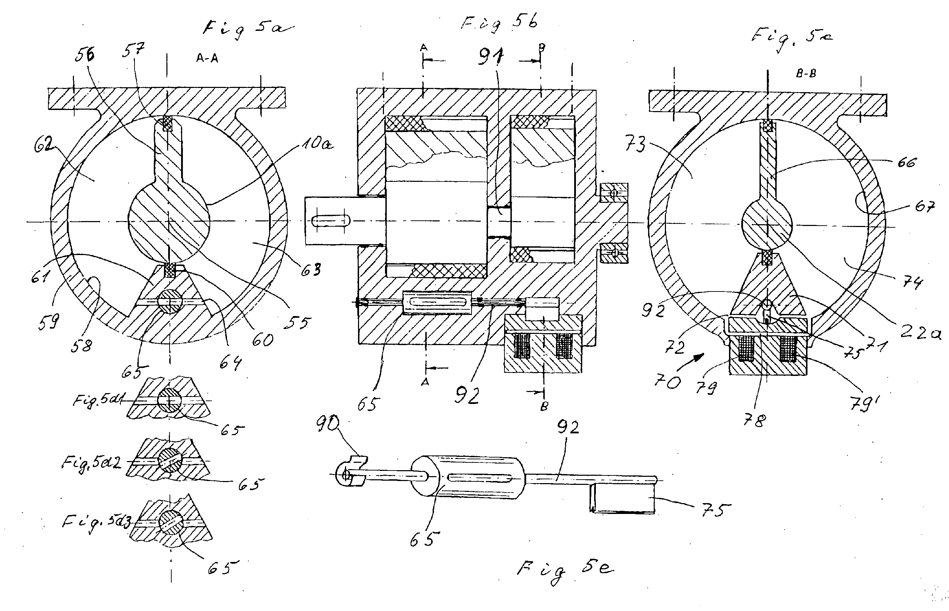

Der Aufbau des Bewegungsdämpfers mit Dämpfungszylinder und damit axial bewegten Dämpfungskolben, wie er in den Figuren 3 und 4 dargestellt ist, kann auch mit Drehkolben erfolgen, die sich verdrehend in einem Dämpfungszylinder bewegen. Dies ist in dem Ausführungsbeispiel gemäß den Figuren 5a bis e dargestellt. Dabei zeigt die Fig. 5a einen Schnitt längs der Linie A-A aus Fig. 5b und die Fig. 5c einen Schnitt längs der Linie B-B aus der Fig. 5b.

Bei dem Ausführungsbeispiel gemäß Fig. 5a ist der Dämpfungskolben als Drehkolben 10a ausgebildet, der sich um die Achse 55 dreht. Der Drehkolben 10a besitzt den Flügel 56, der sich über die Dichtung 57 an der Innenwand 58 des Dämpfungszylinders 59 dichtend abstützt. Auf seiner dem Dämpfungsflügel 56 abgewandten Seite hält der Drehkolben 10a dichtenden Kontakt mit der Dichtung 60, die in die sektorial verlaufende Wand 58 des Dämpfungszylinders 59 eingelassen ist. Damit sind in dem Dämpfungszylinder 59 die beiden Dämpfüngsräume 62 und 63 ausgebildet, die durch den Drehkolben 10a mit dem Dämpfungsflügel 56 voneinander getrennt sind.

Die beiden Dämpfungsräume 62 und 63 sind durch den Verbindungskanal 64 miteinander verbunden, in dem das Dämpfungsventil 65 drehbar angebracht ist, auf dessen Wirkungsweise im Zusammenhang mit den Figuren 5d1, 5d2 und 5d3 näher eingegangen wird. Bei der in der Figur 5a dargestellten Durchlässigkeitsstellung des Dämpfungsventils 65 kann die in den beiden Dämpfungsräumen 62 und 63 enthaltene Dämpfungsflüssigkeit praktisch ungehindert von dem einen Dämpfungsraum 62 in den anderen Dämpfungsraum 63 und umgekehrt strömen.

Mit dem Drehkolben 10a ist koaxial die Schubkolbeneinheit 70 mit dem Schubkolben 22a verbunden (siehe Fig. 5c), der wie der Drehkolben 10a gemäß Fig. 5a ebenfalls als Drehkolben ausgebildet ist und bei der Verdrehung des Drehkolbens 10a mitverdreht wird. Der Schubkolben 22a besitzt wie der Drehkolben 10a gemäß Fig. 5a den Flügel 66, der sich bei der Verdrehung des Schubkolbens 22a mitverdreht. Die Abdichtung des Schubkolbens 22a und des Flügels 66 gegenüber der Innenwand 67 der Schubkolbeneinheit gemäß Fig. 5c erfolgt dabei in gleicher Weise, wie dies oben im Zusammenhang mit dem Drehkolben 10a beschrieben ist.

Der Bewegungsdämpfer gemäß den Figuren 5a bis 5e wird nun bei der Beugung einer Beinprothese in einer Weise betätigt, die der Betätigung der Bewegungsdämpfer gemäß den Figuren 3 und 4 entspricht, d.h., es erfolgt eine Verdrehung des Drehkolbens 10a mit dem Beugen des Kniegelenks der Prothese, womit auch der Schubkolben 22a mit seinem Flügel 66 gemäß Fig. 5c verdreht wird, da der Drehkolben 10a und der Schubkolben 22a über das in Fig. 5b dargestellte Verbindungsstück 91 miteinander drehfest verbunden sind.

Ähnlich wie bei der Gestaltung gemäß Fig. 5a ragt in den Innenraum des Schubkolbenzylinders 70 die Wand 71 hinein, die eine Verbindung 72 zwischen den beiden Stellräumen 73 und 74 enthält. In diese Verbindung 72 ist ein Drehflügel 75 eingebaut, der von einem auf seiner einen Seite wirkenden Druck entsprechend verdreht wird. Die den Schubkolben 22a enthaltende Schubkolbeneinheit 70 ist mit magnetorheologischer Flüssigkeit gefüllt, die bei Verdrehung des Flügels 66 von dem einen Stellraum 73 in den anderen Stellraum 74 über die Verbindung 72 verschoben wird und dabei den Drehflügel 75 entsprechend mitnimmt. Dieser Mitnahme setzt der Drehflügel 75 durch eine entsprechend eingestellte Drehfeder (90) einen Widerstand entgegen, ähnlich wie dies bei der Zwischenwand 31 gemäß Fig. 3 der Fall ist. Der Verbindung 72 ist nun strömungsmäßig der Nebenschluss 78 parallel geschaltet, der bei Durchlässigkeit eine Strömung der magnetorheologischen Flüssigkeit von dem einen Stellraum 73 in den anderen Stellraum 74 bzw. umgekehrt praktisch ungehindert zulässt. In diesem Nebenschluss 78 sind nun die beiden Magnetfelderzeuger 79 und 79' eingebaut, die durch einen entsprechend eingestellten Stromfluss mit dem von ihnen erzeugten Magnetfeld den Durchfluss durch den Nebenschluss 78 steuern. Die Magnetfelderzeuger 79 und 79' können als Ringpolmagnet ausgebildet sein. Je mehr der Durchfluss der magnetorheologischen Flüssigkeit durch den Nebenschluss 78 durch die Magnetfelderzeuger 79 und 79' gehindert wird, um so mehr wirkt sich der Druckunterschied in den beiden Stellräumen 73 und 74 aus, was sich in einer entsprechenden Verstellung des Drehflügels 75 äußert. Die Achse 92 des Drehflügels 75 ist nun, wie die Fig. 5e zeigt, direkt mit dem Dämpfungsventil 65 verbunden, das bei seiner Verdrehung, wie die Figuren 5d1, 5d2 und 5d3 deutlich zeigen, den Durchfluss der den Verbindungskanal 64 durchströmenden Dämpfungsflüssigkeit mehr oder minder behindert und damit eine entsprechende Bedämpfung der Drehbewegung des Flügels 56 und damit des Dämpfungskolbens 10a bewirkt.

In der Fig. 5e ist die Verbindung zwischen dem Drehflügel 75 und dem Dämpfungsventil 65 über die gemeinsame Achse 92 verdeutlicht.

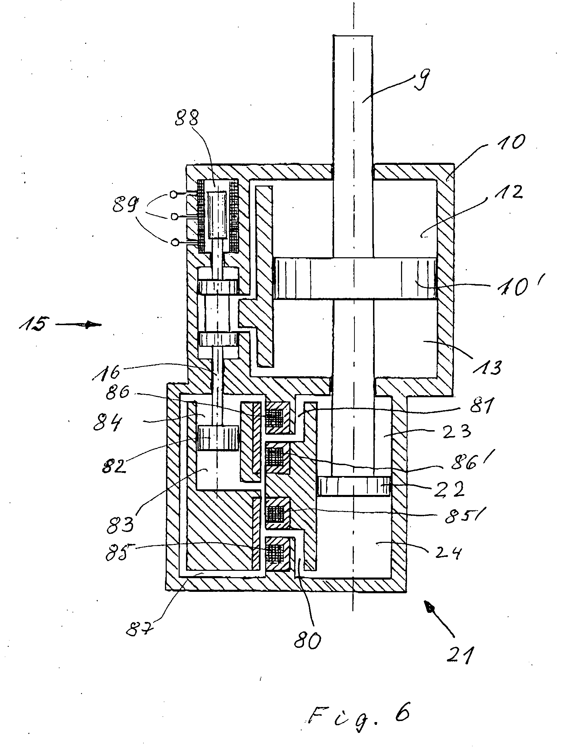

Mit den vorstehend dargestellten Ausführungsbeispielen eines regelbaren Bewegungsdämpfers wird die Bewegung des Dämpfungskolbens mit jedem Hub in beiderlei Richtung je nach Stärke des eingestellten Magnetfeldes über die magnetorheologische Flüssigkeit bedämpft. Mit der Beendigung eines Hubes endet damit auch die Dämpfungswirkung. Es ist nun auch möglich, die Dämpfungswirkung dauernd fest auf einen Wert einzustellen, der durch die Erregung des betreffenden Magnetfelderzeugers eingestellt wird. Ein Ausführungsbeispiel hierfür zeigt die Fig. 6, die hinsichtlich des Dämpfungskolbens mit Dämpfungszylinder und dem Dämpfungsventil der Gestaltung gemäß Fig. 3 entspricht. Die Bewegung des Dämpfungsventils 15 (gleiche Bezeichnung wie in Fig. 3) erfolgt allerdings in dem Ausführungsbeispiel gemäß Fig. 6 in folgender Weise:

Das Dämpfungsventil 15 wird über den Stößel 16 verstellt und ändert damit, wie dies beim Ausführungsbeispiel gemäß Fig. 3 beschrieben ist, seine Stellung und die Bedämpfung des Dämpfungskolbenstößels 9. Die Energie für die Verschiebung des Stößels 16 wird, wie beim Ausführungsbeispiel gemäß Fig. 3, von der Bewegung des Dämpfungskolbenstößels 9 abgezweigt, der bei seiner Bewegung und der des Schubkolbens 22 in den Kanälen 80 und 81 eine magnetorheologische Flüssigkeit verschiebt, die, wie beim Ausführungsbeispiel gemäß Fig. 3 gezeigt, den Bereich mit der Schubkolbeneinheit 21 ausfüllt. Die Kanäle 80 und 81 reichen in die von dem Steuerkolben 82 getrennten Räume 83 und 84, d.h., ein Druck im Raum 83 bzw. 84 bewirkt eine entsprechende axiale Verschiebung des Steuerkolbens 82. Dieser Druck auf den jeweiligen Raum 83 bzw. 84 wird nun über die Magnetfelderzeuger 85 und 85' sowie 86 und 86' gesteuert, die jeweils auf die Verbindung vom Kanal 80 bzw. 81 zu dem Raum 83 bzw. 84 wirken und somit je nach Stärke ihres Magnetfeldes die Verschiebung des Steuerkolbens 82 entsprechend beeinflussen. Dabei ist der Kanal 80 über die Verbindung 87 an den Raum 84 angeschlossen. Damit besteht die Möglichkeit, über jeden der beiden Kanäle 80 und 81 auf die jeweils gewünschte Seite des Steuerkolbens 82 einzuwirken, womit dieser willkürlich entsprechend der Steuerung der Magnetfelderzeuger verschoben wird. Mit dieser Verschiebung des Steuerkolbens 82 wird über den Stößel 16 das Dämpfungsventil 15 entsprechend eingestellt, womit sich eine fest eingestellte Bedämpfung des Dämpfungskolbens 9 ergibt.

Diese Einstellung der Bedämpfung wird über den an den Stößel 16 und das Dämpfungsventil 15 angeschlossenen Sensor 88 angegeben, der einen in bekannter Weise aufgebauten Magnetsensor enthält, der je nach seiner Einstellung verschiedene Organe, aktiviert, die die Einstellung des Dämpfungsventils 15 wiedergeben und einer Steuerung mitteilen.

### Bezugszeichenliste

- 1: Kniegelenk
- 2: Achse
- 3: Oberes Unterschenkelteil
- 4: Bewegungsdämpfer
- 5: Fußteil
- 6: Unteres Unterschenkelteil
- 7: Oberschenkelteil
- 8: Hebelarm
- 9: Dämpfungskolbenstößel
- 10: Dämpfungszylinder
- 10': Dämpfungskolben
- 11: Achse / Dämpfungskolbenteller
- 12: Dämpfungsraum
- 13: Dämpfungsraum
- 15: Dämpfungsventil
- 16: Stößel
- 17: Ventilteller
- 18: Ventilteller
- 19: Dämpfungsventilzylinder
- 20: Zwischenraum
- 21: Schubkolbeneinheit
- 22: Schubkolben
- 23: Stellräume
- 24: Stellräume
- 25: Nebenschluss
- 26: Nebenschlussteile
- 27: Nebenschlussteile
- 28: Kanalteil
- 29: Kanalteil
- 30: Magnetfelderzeuger
- 31: Zwischenwand
- 34: Rohrstück
- 36: Öffnungen
- 37: Öffnungen
- 38: Hohlraum
- 39: Ventilteil
- 40: Stößel
- 41: Zwischenwand
- 42: Räume
- 43: Räume
- 44: Zylinder
- 45: Schubkolben
- 46: Nebenschlussteil
- 47: Nebenschlussteil
- 48: Magnetfelderzeuger
- 49: Magnetfelderzeuger
- 55: Achse
- 56: Dämpfungsflügel
- 57: Dichtung
- 58: Innenwand
- 59: Dämpfungszylinder
- 60: Dichtung
- 62: Dämpfungsraum
- 63: Dämpfungsraum
- 64: Verbindungskanal
- 65: Dämpfungsventil
- 66: Flügel
- 67: Innenwand
- 70: Schubkolbenzylinder
- 71: Wand
- 72: Verbindung
- 73: Stellraum
- 74: Stellraum
- 75: Drehflügel
- 78: Nebenschluss
- 79: Magnetfelderzeuger
- 79': Magnetfelderzeuger
- 80: Kanal
- 81: Kanal
- 82: Steuerkolben
- 83: Raum
- 84: Raum
- 85: Magnetfelderzeuger
- 86: Magnetfelderzeuger
- 87: Verbindung
- 88: Sensor
- 89: Leuchtdioden
- 90: Drehfeder
- 91: Verbindungsstück
- 92: Achse

## Patentansprüche

1. Regelbarer Bewegungsdämpfer (4) mit einem Dämpfungskolben (10'), der in einem Dämpfungszylinder (10) zwei Dämpfungsräume (12, 13) gegeneinander abgrenzt, und einem in einem Verbindungskanal (28, 29) der beiden Dämpfungsräume (12, 13) angeordneten Dämpfungsventil (15), dessen Durchlässigkeit für ein im Bewegungsdämpfer (4) enthaltenes Dämpfungsfluid von einem Steuerorgan (21, 16) gesteuert wird, **dadurch gekennzeichnet, dass** beide Dämpfungsräume (12, 13) mit einem elektromagnetisch unbeeinflussbaren Dämpfungsfluid gefüllt sind und das Steuerorgan eine mit einer magnetorheologischen Flüssigkeit gefüllte Schubkolbeneinheit (21) mit einem kraftschlüssig mit dem Dämpfungskolben (10') gekoppelten Schubkolben (22) aufweist, der über die durch steuerbare Magnetfelderzeuger (30) hinsichtlich ihres Strömungswiderstandes beeinflussbare magnetorheologische Flüssigkeit auf ein Stellglied (16) wirkt, das mit dem Dämpfungsventil (15) verbunden ist.

2. Bewegungsdämpfer nach Anspruch 1, **dadurch gekennzeichnet, dass** die Schubkolbeneinheit (21) zwei durch den Schubkolben (22) getrennte Stellräume (23, 24) mit einem diese verbindenden Nebenschluss (26, 27) enthält, dessen Strömungswiderstand durch die Magnetfelderzeuger (30) einstellbar ist.

3. Bewegungsdämpfer nach Anspruch 2, **dadurch gekennzeichnet, dass** das Dämpfungsventil (15) mit einem das Stellglied bildenden, von einer Rückstellfeder geführten Stößel (16) verbunden ist, der mit einer Zwischenwand (31) verstellbar verbunden ist, die die Schubkolbeneinheit (21) in die zwei über den Nebenschluss (26, 27) verbundenen Stellräume (23, 24) aufteilt.

4. Bewegungsdämpfer nach einem der Ansprüche 1 bis 3, **dadurch gekennzeichnet, dass** das Dämpfungsventil (15) neben dem Dämpfungszylinder (10) und die Schubkolbeneinheit (21) koaxial zum Dämpfungszylinder (10) angeordnet ist.

5. Bewegungsdämpfer nach einem der Ansprüche 1 bis 3, **dadurch gekennzeichnet, dass** das Dämpfungsventil (38, 39) koaxial zum Dämpfungskolben (10') als Bestandteil des Dämpfungskolbens (10') angeordnet ist.

6. Bewegungsdämpfer nach Anspruch 1 oder 2, **dadurch gekennzeichnet, dass** der Bewegungsdämpfer und die Schubkolbeneinheit jeweils als Drehkolben (10a, 22a) gestaltet sind und in jeweils einer sektorial verlaufenden Wand (61, 71) das Dämpfungsventil (65) und der Nebenschluss (72, 78) vorgesehen sind, wobei das Dämpfungsventil als Drehventil (65) ausgebildet ist und der Nebenschluss (72) einen Drehflügel (75) als Bestandteil des Stellglieds enthält und Drehventil (65) und Drehflügel (75) über eine Achse (92) miteinander verbunden und durch eine Drehfeder (90) in Normallage gehalten sind.

7. Bewegungsdämpfer nach Anspruch 1 oder 2, **dadurch gekennzeichnet, dass** das Dämpfungsventil (15) über einen Steuerkolben (82) mit dem Stellglied (16) verbunden ist, das mit der Schubkolbeneinheit (21) über mehrere jeweils durch Magnetfelderzeuger (85, 86) einstellbare Nebenschlüsse (80, 81) verbunden ist, die in Abhängigkeit von der Aktivierung der einzelnen Magnetfelderzeuger (85, 86) den Steuerkolben (82) in beliebige Stellungen, insbesondere der Bewegung des Schubkolbens (22) folgend, einstellen.

8. Bewegungsdämpfer nach einem der Ansprüche 1 bis 3, **dadurch gekennzeichnet, dass** die verschiedenen Bewegungsdämpfer anstelle mit magnetorheologischer Flüssigkeit mit elektrorheologischer Flüssigkeit versehen sind.
